# EUROPEAN PATENT SPECIFICATION

(11) **EP 4 499 158 B1**
(45) Date of publication and mention of the grant of the patent: **11.02.2026**
(21) Application number: 23717643.3
(22) Date of filing: 21.03.2023
(51) Int. Cl.: A61L 9/12, B60N 2/00, B60H 3/00, B60H 1/00, B60N 2/879

(54) **TEXTILE ELEMENT WITH PASSIVE FRAGRANCE DIFFUSER**
TEXTILELEMENT MIT PASSIVEM DUFTDIFFUSOR
ÉLÉMENT TEXTILE COMPRENANT UN DIFFUSEUR DE PARFUM PASSIF

(30) Priority: 24.03.2022 IT 202200005894
(43) Date of publication of application: 05.02.2025
(73) Proprietor: Fueguia S.r.l., 20127 Milano (MI) (IT)
(72) Inventor: BEDEL, Julian Francisco, 20127 Milano MI (IT)
(74) Representative: Perani & Partners S.p.A.
(86) International application number: PCT/IB2023/052764
(87) International publication number: WO 2023/180927

(56) References cited:
- GB-A- 2 586 630
- US-A- 5 782 409
- US-A1- 2014 028 063
- US-B1- 9 272 062

## Description

### FIELD OF APPLICATION

The present invention relates to a textile element comprising a passive fragrance diffuser. **In** particular, the textile element is a headrest cushion for vehicles such as cars, aircraft, trains and boats, into which such a diffuser may be inserted.

### Description of the prior art

It is known in the art to manufacture a disposable passive fragrance diffuser consisting of a material impregnated with a fragrance. Known diffusers are used in cars or enclosed spaces to diffuse the fragrance into the environment in direct contact with the air.

They are also known passive fragrance diffusers comprising a fabric bag containing therein fragrance granules diffusing the fragrance into the surrounding environment through direct contact with the air.

Known passive fragrance diffusers are disclosed in US 5782409 A, WO 99/06075 A1 and WO 2018/067621 A1, US 2014/028063 A, GB 2 586 630 A and US 5782409 A.

### Problem of the prior art

In the prior art, the diffusion of the fragrance occurs by direct contact with the air. This results in a continuous and totally unpredictable release of the fragrance. Indeed, the environmental ventilation, humidity and temperature conditions, as well as agents such as water or sunlight, have a decisive impact on how the fragrance is released and on the durability of the diffuser before the fragrance is used up. In addition, the durability of the diffuser is also reduced by direct contact with the air, which results in a continuous release of the fragrance, even when it is not necessary, i.e. when there is nobody to benefit from it.

### SUMMARY OF THE INVENTION

A further object of the invention is to use a textile element comprising a passive fragrance diffuser which is able to overcome the drawbacks of the prior art.

A further object of the invention is to use a textile element in the form of a headrest cushion for vehicles comprising a passive fragrance diffuser, which is able to overcome the drawbacks of the prior art.

The defined technical task and the specified objects are substantially achieved by a textile element comprising the technical characteristics set forth in one or more of the appended claims.

### Advantages of the invention

Thanks to an embodiment, it is possible to obtain a textile element that makes it possible to extend the durability of the diffuser incorporated therein.

Thanks to an embodiment, it is possible to create a textile element that makes it possible to release the fragrance in a controlled manner.

Thanks to an embodiment, it is possible to produce a textile element, and in particular a headrest cushion for vehicles, comprising a passive fragrance diffuser that makes it possible to release the fragrance in a controlled manner, only when necessary.

### BRIEF DESCRIPTION OF THE DRA WINGS

The characteristics and advantages of the present invention will become clear from the following detailed description of a possible practical embodiment, illustrated by way of a non-limiting example in the set of drawings, wherein:
- Figure 1 shows a front view of a passive fragrance diffuser according to the present invention,
- Figure 2 shows a transversal section view of the diffuser of Figure 1,
- Figure 3 shows a detail of the diffuser shown in Figure 2.
- Figure 4 shows a textile element in the form of a headrest cushion for vehicles, provided with the diffuser shown in Figure 1.

### DETAILED DESCRIPTION

The present invention relates to a textile element provided with a passive fragrance diffuser 1.

The diffuser 1 comprises an inner layer 2 imbued with a fragrance. The inner layer 2 is provided with a peripheral rim B, which defines the perimeter thereof, being preferably a layer with a substantially planar extension, more preferably with a perfectly planar extension having an even thickness.

The diffuser 1 comprises a first outer layer 3 which is hydrophobic, oleophobic and permeable to air. The diffuser 1 also comprises a second outer layer 4 which is hydrophobic, oleophobic and permeable to air. The inner layer 2 is enclosed between the first outer layer 3 and the second outer layer 4. Preferably, the first outer layer 3 and the second outer layer 4 are identical, e.g. two layers made of the same material and of the same dimensions.

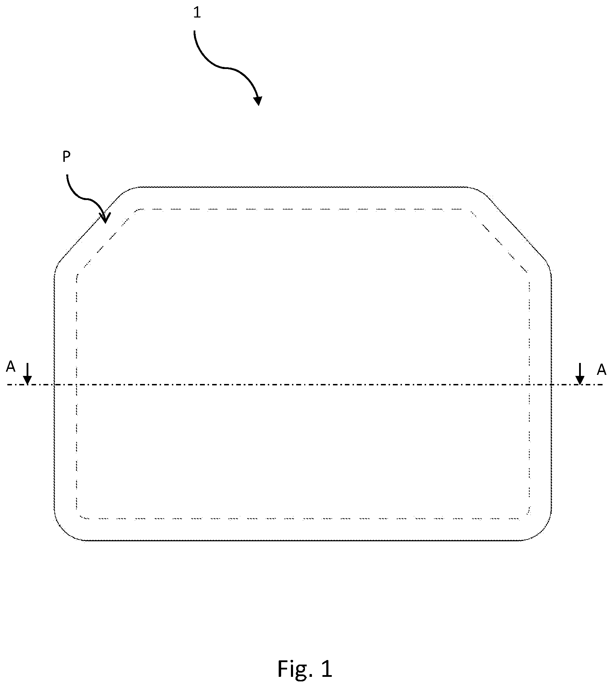

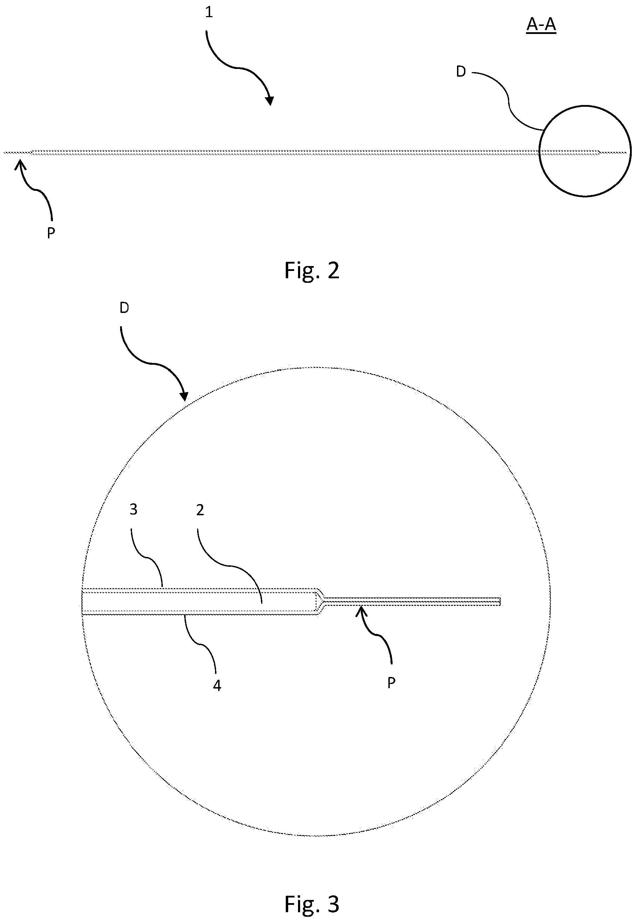

The first outer layer 3 and the second outer layer 4 are joined together by a double-sided adhesive tape at least along a peripheral area P surrounding the peripheral rim B of the inner layer 2 (see Figures 1-3). Alternatively, the first outer layer 3 and the second outer layer 4 are joined together by casting or welding, e.g. by ultrasonic welding.

It should be noted that the diffuser 1 is a passive multilayer system for diffusing a fragrance by evaporation.

According to a preferred embodiment of the invention, the inner layer 2 is made of absorbent material with high liquid retention capacity. Preferably, the inner layer 2 is made of porous material. Still preferably, the porous material may contain up to ten times its weight of liquid without dripping. Even more preferably, the material of the inner layer 2 comprises polyester PE and/or polypropylene PP fibre and/or polyvinyl acetate PVA. still preferably with a thickness of 1.5 ± 0.5 mm. Advantageously, this material offers a high liquid retention capacity without reaching saturation. Advantageously, the inner layer 2 behaves like a sponge, with a porosity that determines the speed of diffusion of the fragrance and thus the durability of the diffuser 1 itself, i.e. the length of time the diffuser 1 is able to diffuse the fragrance before it is used up. It should be noted that the absorbent material of the inner layer 2 can be natural, synthetic or mixed, preferably flexible but not necessarily porous. Moreover, its characteristics and properties make it possible to release in a controlled way and slowly the fragrance with which the inner layer 2 is impregnated.

According to a preferred embodiment, the first outer layer 3 and the second outer layer 4 are made of polymeric material, preferably PTFE with a thickness of 0.13 mm. Preferably, the first outer layer 3 and the second outer layer 4 are made of GORE-TEX, i.e. an extremely thin layer/membrane of expanded polytetrafluoroethylene (ePTFE) that controls the diffusion of the fragrance through the passage of air (porosity) and thus the product durability, water-resistance and breathability. Advantageously, this material ensures water-resistance and fragrance release capacity, without releasing essential oils or solvents. According to a preferred embodiment, the inner layer 2 of polyester and/or polypropylene is then trapped between two outer layers 3, 4 of PTFE, which by capillarity and surface tension, retain the perfume. The outer layers 2, 3 of PTFE are very thin with a very low porosity, of about half a micron. Porosity allows control over how the fragrance is released, allowing air to pass through but not the liquid fragrance impregnated in the inner layer 2.

According to a preferred embodiment, the double-sided adhesive tape comprises a central polymer layer enclosed between two layers of acrylic adhesive, and has an overall thickness of 0.20 mm. Advantageously, the double-sided adhesive tape allows the outer layers 3, 4 to be firmly joined.

When the diffuser 1 is produced, the fragrance (or perfume) is prepared in a solution with a solvent suitable for room diffusers and applied to the inner layer 2, which is then enclosed and sealed between the two outer layers 3, 4 with double-sided adhesive tape.

The textile element is provided with a pocket T within which the diffuser 1 is removably inserted. Preferably the textile element is a cushion such as a pillow, or a garment, provided with a pocket T into which the diffuser 1 is inserted. Preferably, the pocket T should have a closable mouth, e.g. by means of zips, magnets, fabric flaps, Velcro, buttons or other known systems. Other application examples are textile elements for indoor environments such as homes/hotels/offices/restaurants, e.g. mattress padding, sofas, cushions, chairs, headboards, wardrobes and drawers. Other examples of textile elements are bags, suitcases and backpacks. Preferably, the textile element comprises a textile portion including the pocket T, which is made of breathable textile material to allow the diffusion of the fragrance from the diffuser 1 to the outside.

According to a preferred embodiment, the passive fragrance diffuser 1 comprises at least one coupling member, preferably releasable, such as, for example, Velcro or a magnet or a coupling/uncoupling member, for coupling inside the pocket T, in which corresponding coupling members are preferably present.

Always preferably, the textile element includes coupling means, more preferably releasable, such as Velcro or a magnet or a coupling/uncoupling member, for fastening to corresponding outer coupling means.

Advantageously, the textile element may be positioned close to the air vent of a vehicle such as a car, e.g. even under the seat of the car, aircraft or vessel. Advantageously, the diffuser 1 contained in the textile element can diffuse the fragrance with the help of the air coming out of the nozzle, or to other parts of the car where the fragrance can also diffuse thanks to air circulation.

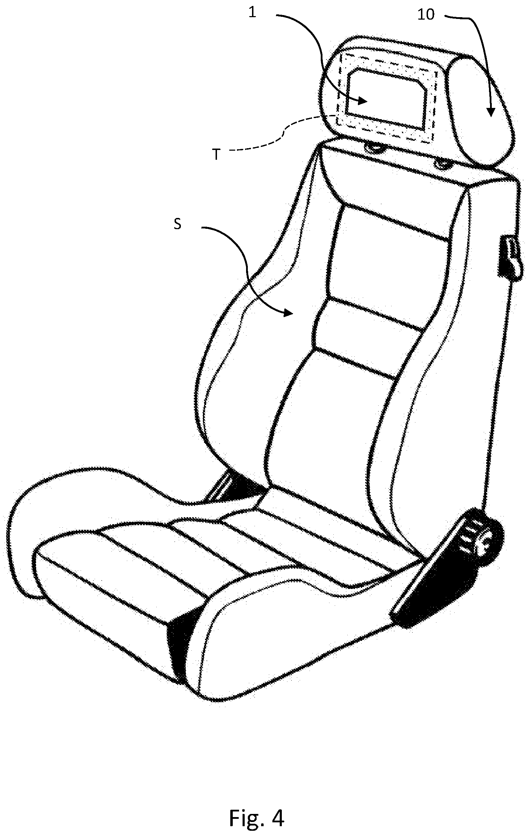

Referring to Figure 4, in the preferred embodiment of the present invention, the textile element is a vehicle headrest cushion 10 of the type found on the seat S of a passenger car, aircraft, train or vessel, provided with an inner pocket T and comprising a passive fragrance diffuser 1 removably inserted within the pocket T. It should be noted herein that the trapezoidal shape and proportions of the diffuser 1 shown in the appended figures are suitable for this specific application. Advantageously, the diffuser 1 releases the fragrance when the person rests their head on the headrest cushion, as the air movement thus caused in the cushion facilitates the diffusion of the fragrance.

The inner pocket T should preferably have a closable mouth, e.g. by means of zips, magnets, fabric flaps, Velcro, buttons or other known systems. Advantageously, the diffuser 1 may be removed and replaced by inserting a new diffuser 1 into the pocket T when the fragrance has been used up. As the diffuser 1 is a disposable product, it can be produced to last between six months and one year by controlling the properties of the layers 2, 3, 4 of the diffuser 1. Advantageously, the headrest cushion 10 for vehicles can be used in cars, planes, boats and trains.

It should be noted that the durability of the diffuser depends on where it is applied, i.e. the space where it is placed and the size and shape thereof.

Advantageously, the diffuser 1 may be removed and replaced with a new diffuser 1 to be inserted into the pocket T when the fragrance has been used up in the previous diffuser.

## Claims

1. Textile element **characterized in that** it comprises:
- a passive fragrance diffuser (1), comprising:
- an inner layer (2) imbued with a fragrance and provided with a peripheral rim (B);
- a first outer layer (3) which is hydrophobic, oleophobic and permeable to air;
- a second outer layer (4) which is hydrophobic, oleophobic and permeable to air;
wherein
- the inner layer (2) is enclosed between the first outer layer (3) and the second outer layer (4),
- the first outer layer (3) and the second outer layer (4) are joined together by a double-sided adhesive tape at least along a peripheral area (P) surrounding the peripheral rim (B) of the inner layer (2);
- a pocket (T) inside which the diffuser (1) is removably inserted.

2. Textile element according to claim 1, wherein the inner layer (2) is made of absorbent material with high liquid retention capacity.

3. Textile element according to claim 2, wherein the inner layer (2) is made of porous material.

4. Textile element according to claim 2 or 3, wherein the material of the inner layer (2) comprises polyethylene and/or polypropylene, preferably with a thickness of 1.5 ± 0.5 mm.

5. Textile element according to any one of the preceding claims, wherein the first outer layer (3) and the second outer layer (4) are made of polymeric material, preferably PTFE with a thickness of 0.13 mm.

6. Textile element according to any one of the preceding claims, wherein the double-sided tape comprises a central polymeric layer comprised between two layers of acrylic adhesive.

7. Textile element according to any one of the preceding claims, wherein the diffuser (1) comprises at least one coupling member for coupling inside the pocket (T).

8. Textile element according to claim 7, wherein the coupling member comprises Velcro or a magnet.

9. Textile element according to any of the previous claims, wherein the pocket (T) is provided with a closable mouth.

10. Textile element according to any of the previous claims, wherein the textile element is a headrest cushion (10) for vehicles.

## Patentansprüche

1. Textilelement, **dadurch gekennzeichnet, dass** es umfasst:
∘ einen passiven Duftdiffusor (1), umfassend:
▪ eine innere Schicht (2), die mit einem Duftstoff getränkt und mit einem Umfangsrand (B) versehen ist;
▪ eine erste äußere Schicht (3), die hydrophob, oleophob und luftdurchlässig ist;
▪ eine zweite äußere Schicht (4), die hydrophob, oleophob und luftdurchlässig ist; wobei
∘ die innere Schicht (2) zwischen der ersten äußeren Schicht (3) und der zweiten äußeren Schicht (4) eingeschlossen ist,
∘ die erste äußere Schicht (3) und die zweite äußere Schicht (4) durch ein doppelseitiges Klebeband mindestens entlang eines Umfangsbereichs (P), der den Umfangsrand (B) der inneren Schicht (2) umgibt, miteinander verbunden sind;
∘ eine Tasche (T), in die der Diffusor (1) entfernbar eingesetzt ist.

2. Textilelement nach Anspruch 1, wobei die innere Schicht (2) aus saugfähigem Material mit hoher Flüssigkeitsrückhaltekapazität hergestellt ist.

3. Textilelement nach Anspruch 2, wobei die innere Schicht (2) aus porösem Material hergestellt ist.

4. Textilelement nach Anspruch 2 oder 3, wobei das Material der inneren Schicht (2) Polyethylen und/oder Polypropylen umfasst, vorzugsweise mit einer Dicke von 1,5 ± 0,5 mm.

5. Textilelement nach einem der vorhergehenden Ansprüche, wobei die erste äußere Schicht (3) und die zweite äußere Schicht (4) aus Polymermaterial, vorzugsweise PTFE mit einer Dicke von 0,13 mm, hergestellt sind.

6. Textilelement nach einem der vorhergehenden Ansprüche, wobei das doppelseitige Klebeband eine zentrale Polymerschicht umfasst, die zwischen zwei Schichten aus Acrylklebstoff eingeschlossen ist.

7. Textilelement nach einem der vorhergehenden Ansprüche, wobei der Diffusor (1) mindestens ein Kopplungselement zum Koppeln innerhalb der Tasche (T) umfasst.

8. Textilelement nach Anspruch 7, wobei das Kopplungselement einen Klettverschluss oder einen Magneten umfasst.

9. Textilelement nach einem der vorhergehenden Ansprüche, wobei die Tasche (T) mit einer verschließbaren Öffnung versehen ist.

10. Textilelement nach einem der vorhergehenden Ansprüche, wobei das Textilelement ein Kopfstützenkissen (10) für Fahrzeuge ist.

## Revendications

1. Élément textile **caractérisé en ce que** il comprend:
∘ un diffuseur de parfum passif (1), comprenant:
▪ une couche intérieure (2) imprégnée d'un parfum et pourvue d'un bord périphérique (B);
▪ une première couche extérieure (3) qui est hydrophobe, oléophobe et perméable à l'air;
▪ une seconde couche extérieure (4) qui est hydrophobe, oléophobe et perméable à l'air; dans lequel
▪ la couche intérieure (2) est enfermée entre la première couche extérieure (3) et la seconde couche extérieure (4),
▪ la première couche extérieure (3) et la seconde couche extérieure (4) sont solidarisées par un ruban adhésif double face au moins le long d'une zone périphérique (P) entourant le bord périphérique (B) de la couche intérieure (2);
∘ une poche (T) à l'intérieur de laquelle le diffuseur (1) est inséré de manière amovible.

2. Élément textile selon la revendication 1, dans lequel la couche intérieure (2) est faite d'un matériau absorbant ayant une capacité de rétention de liquide élevée.

3. Élément textile selon la revendication 2, dans lequel la couche intérieure (2) est faite d'un matériau poreux.

4. Élément textile selon la revendication 2 ou 3, dans lequel le matériau de la couche intérieure (2) comprend du polyéthylène et/ou du polypropylène, de préférence avec une épaisseur de 1,5 ± 0,5 mm.

5. Élément textile selon l'une quelconque des revendications précédentes, dans lequel la première couche extérieure (3) et la seconde couche extérieure (4) sont faites d'un matériau polymère, de préférence du PTFE avec une épaisseur de 0,13 mm.

6. Élément textile selon l'une quelconque des revendications précédentes, dans lequel le ruban adhésif double face comprend une couche polymère centrale comprise entre deux couches d'adhésif acrylique.

7. Élément textile selon l'une quelconque des revendications précédentes, dans lequel le diffuseur (1) comprend au moins un élément d'accouplement pour l'accouplement à l'intérieur de la poche (T).

8. Élément textile selon la revendication 7, dans lequel l'élément d'accouplement comprend du Velcro ou un aimant.

9. Élément textile selon l'une quelconque des revendications précédentes, dans lequel la poche (T) est pourvue d'une ouverture refermable.

10. Élément textile selon l'une quelconque des revendications précédentes, dans lequel l'élément textile est un coussin d'appui-tête (10) pour véhicules.
